# EUROPEAN PATENT APPLICATION

(11) **EP 4 282 404 A1**
(43) Date of publication of application: **29.11.2023**
(21) Application number: 22175921.0
(22) Date of filing: 27.05.2022
(51) Int. Cl.: A61K 9/10, A61K 9/127, A61K 31/00

(54) **METHOD FOR PREPARING NANOSYSTEMS ENCAPSULATING ONE OR MORE ACTIVE INGREDIENTS**

(71) Applicant: Université de Genève, 1211 Genève 4 (CH)
(72) Inventor: Kalia, Yogeshvar N., 1232 Confignon (CH); Darade, Aditya Ramnath, 1206 Geneve (CH)
(74) Representative: Casalonga

(57) **Abstract**

The present invention deals with a method for preparing a nanosystem vehicle encapsulating one or more active ingredients, preferably selected from pharmaceutical active ingredients, subsequently comprising cryomilling one or more amphiphilic compounds, one or more of said active ingredients and a frozen aqueous medium in order to obtain a uniform frozen mixture, and forming said nanosystem vehicle upon thawing.

The invention further relates to a nanosystem vehicle obtainable by the aforementioned method and its uses for therapeutic and non-therapeutic applications.

## Description

### Field of the invention

The present invention deals with a method for preparing a nanosystem vehicle encapsulating one or more active ingredients, preferably selected from pharmaceutical active ingredients, subsequently comprising cryomilling one or more amphiphilic compounds, one or more of said active ingredients and a frozen aqueous medium in order to obtain a uniform frozen mixture, and forming said nanosystem vehicle upon thawing.

The invention further relates to a nanosystem vehicle obtainable by the aforementioned method and its uses for therapeutic and non-therapeutic applications.

### Background of the invention

Nanosystem vehicles, especially vesicular nanosystems, are currently often used in therapeutic applications, such as for encapsulating and delivering biological active ingredients, for example drugs, at a controlled rate directly to the targeted sites in the body, e.g. inflamed tissues and/or organs, in order to provide an efficient and adequate therapeutic action over the entire period of treatment, or for stabilizing said biological active ingredients, such as messenger RNA (mRNA) in gene therapy.

Nanosystem vehicles have also been developed and successfully used in non-therapeutic applications, for example in the preparation of topical formulations intended for cosmetic and/or dermatological purposes. Indeed, nanosystems can help penetrate or absorb cosmetic or dermatologic active ingredients within epidermal layers and/or in the dermis in a controlled manner to achieve burst and/or sustain release of said active ingredients.

Nanosystem vehicles can include monolayer vesicle structures, especially micelles such as polymeric micelles, as well as bilayer vesicle structures, such as liposomes, niosomes, sphingosomes, transferosomes, ethosomes and so forth. These vesicle nanosystems are generally formed by the self-association of amphiphilic compounds when they are added to an aqueous solution. In particular, the active ingredients which may be hydrophilic or hydrophobic can be added during or after the formation of said nanosystems vehicles. In case of active drug loading, the pharmaceutical active ingredient is added during the formation of said vehicles and in case of passive drug loading, the pharmaceutical active ingredient is added after such formation.

Currently available methods for preparing such nanosystem vehicles generally involve the implementation and subsequent removal of organic solvents.

For instance, liposomes are generally made with amphiphilic lipids, e.g. phospholipids that are first dissolved and then mixed in an organic solvent, such as a chloroform/methanol mixture, in order to obtain a homogenous mixture of lipids. After being thoroughly mixed, the organic solvent is then removed by evaporation, for example by using a dry nitrogen or argon stream or by rotary evaporation under reduced pressure, to yield a thin lipid film. The film is then hydrated with an aqueous or saline solution which may contain the active ingredient. The dispersion of phospholipids in the aqueous or saline medium leads to the formation of liposomes.

However, it bespeaks that when such methods are implemented for the bulk production of the aforementioned nanosystem vehicles, considerable amounts of organic solvents may be required and then later be discarded by additional steps which can be tedious to carry out. In particular, the complexity of implementing such additional steps may hinge on the nature of the organic solvents. As a consequence, the use and later removal of organic solvents will cause the costs to soar and lengthen the overall manufacturing process.

Furthermore, the use and subsequent removal of organic solvents can also trigger safety issues and health hazards to the operators in industrial production as they are combustible, extremely inflammable and their inhalation can cause a string of serious and various ailments. Besides, the organic solvents should be transported and stored under standardized conditions, which impose additional stringent conditions and ratchet up the industrial costs of the final formulations.

The removal of the organic solvents can also generate wastes that must be properly treated in order to mitigate potential adverse effects caused to the environment.

In addition, the operations of removing the organic solvents may turn out to be not efficient enough and lead to the presence of residues in nanosystem vehicles that can alter the toxicity and the properties of the obtained formulations.

Especially, the residual presence of organic solvents may impede the stability of the final formulations and/or hinder the incorporation, and therefore the action, of the active ingredients in the nanosystem vehicles.

More precisely, in some cases, the organic solvents may weaken the percentage of inclusion or encapsulation of active ingredients and hamper their activity, especially in case of biological active ingredients, and more especially in case of pharmaceutical active ingredients.

As a consequence, the manufacturing steps carried out to get rid of the organic solvents should be carefully and closely monitored to ensure the absence of residues in the final formulations.

Moreover, in case of active drug loadings, the additional steps that are carried out in an attempt to eliminate organic solvents may also degrade or remove at the same time parts of the active ingredients, which had been loaded. Therefore, drugs can be lost or degraded during conventional manufacturing processes.

In other words, the use and later removal of organic solvents can thus be challenging for bulk production of nanosystems media in terms of controlling the costs, ensuring safety and health issues, and obtaining non-toxic, stable and efficient final nanoformulations.

As a result, it remains a real need to provide a method for producing nanosystem vehicles which is easier to implement for bulk production than the current available methods which is also able to ensure safety conditions, both for the operator and the environment, and to lead to stable and efficient formulations.

Specifically, one of the goals of the present invention is to provide a method that is able to alleviate the several flaws occurring in the current available methods, in particular, the risks of solvent hazards, both for the operator and the environment, the number of complicated manufacturing steps required to remove organic solvents and the presence of impurities that may impede the toxicity and the properties of the obtained nanoformulations.

More specifically, another goal of the present invention is to avoid the use and later removal of organic solvents during the manufacture of a nanosystem vehicle.

### Description of the invention

The present invention namely results from the unexpected findings, by the inventors, that the subsequent implementation of cooling of an aqueous medium, preferably water, until freezing, cryomilling one or more amphiphilic compounds, one or more active ingredients, preferably pharmaceutical active ingredients, and said frozen aqueous medium, and thawing the obtained cryomilled mixture, is able to instantly lead to a nanosystem vehicle encapsulating said active ingredients.

Indeed, the step of cryomilling one or more amphiphilic compounds, one or more active ingredients and one or more aqueous medium in a frozen state, especially ice particles, makes it possible to obtain a solid homogenous mixture which has the advantage of displaying a high surface area. In particular, the rheology and physical features of the amphiphilic compounds and the active ingredients are altered in the cryomilled solid mixture compared to those of the individual components.

When the solid cryomilled mixture is then thawed at room temperature or in a hot water bath, the frozen aqueous medium melts and instantly hydrates the mixture of amphiphilic compounds and active ingredients due to the high surface area causing the immediate formation of nanosystems vehicles encapsulating said active ingredients.

Hence, the method according to the present invention does not require the use of organic solvents, a water-soluble solute or a wetting agent, such as glucose, as the frozen aqueous medium, which is already homogenously distributed in the cryomilled solid mixture, hydrates upon thawing the mixture of amphiphilic compounds and active ingredients in order to produce instantly nanosystems vehicles.

Especially, hydration upon thawing is eased by the temporary alteration of the rheological and physical properties of the amphiphilic compounds and the active ingredients.

The method according to the present invention has the further advantage of immediately leading to stable nanosystems vehicles encapsulating one or more active ingredients, preferably selected from pharmaceutical active ingredients, that are ready to be used in therapeutic or non-therapeutic applications.

In other words, the method according to the present invention does not involve the formation of a solid premix, obtainable from cryomilling a composition comprising one or more amphiphilic compounds, one or more active ingredients and one or more water-soluble solutes, which is then dispersed in an aqueous medium in order to produce nanosystems vehicles.

In addition, the method according to the present invention does not require the use and subsequent removal of organic solvents which makes it easier and cheaper to implement for bulk production on an industrial scale than the current available methods while ensuring safety and healthy conditions for the operator.

Especially, the method of the present invention displays the advantage of not depending on the use of an organic solvent which implies that the risks linked to solvent hazards, both for the operator and the environment, the number of manufacturing steps that could be complicated to carry out in order to remove organic solvents, and the possible residual impurities that may alter the toxicity and the properties of the obtained formulations are preferably avoided unlike the current available methods.

More precisely, the method of the present invention is also able to narrow down the unpredictable variables due to the presence of the organic solvents or linked to the operations designed to get rid of them.

Thus, the present invention also relates to a method for preparing a nanosystem vehicle encapsulating one or more active ingredients, comprising:
i) bringing one or more amphiphilic compounds and one or more active ingredients into contact with an aqueous medium in a frozen state,
ii) cryomilling said amphiphilic compounds, said active ingredients and said aqueous medium to obtain a solid cryomilled mixture,
iii) thawing said solid cryomilled mixture until forming said nanosystem vehicle.

Indeed, the method according to the present invention is able to produce stable nanosystem vehicles, especially stable vesicular nanosystem, encapsulating one or more active ingredients.

In addition, the method of the present invention is able to incorporate a higher percentage of active ingredients than current conventional methods without hampering their activity and stability.

In other words, the activity of the active ingredients is less likely to be degraded in the nanosystems obtained by the aforementioned method of the present invention than by conventional methods. Especially, the activity of biological active ingredients, preferably those that are sensitive to the presence of organic solvents, may be enhanced.

As a consequence, it bespeaks that a treatment based on the administration of such nanosystem vehicles may be more efficient as more active ingredients may be delivered to the targeted sites.

Moreover, the method for preparing a nanosystem vehicle according to the invention can be easily optimized and monitored on a lab-scale as well as on a large-scale production.

The method according to the present invention is further suitable for heat-labile biological active ingredients.

On one hand, the obtained nanosystem vehicle may be used in therapeutic applications, preferably in order to deliver drug substances in the body, especially drug substances to targeted sites in the body.

Hence, another subject-matter of the present invention concerns a nanosystem vehicle obtainable from the aforementioned method, for its use in a therapeutic treatment and/or *in vivo* diagnosis method, comprising one or more biological active ingredients preferably chosen in the group consisting of pharmaceutical active ingredients.

On the other hand, the obtained nanosystem vehicle can also be used for non-therapeutic purposes as well.

Another subject-matter of the present invention concerns the use of a nanosystem vehicle as obtained by the aforementioned method, comprising one or more cosmetic active ingredients, in order to impart cosmetic properties to human keratin materials, preferably to skin.

Other subjects and characteristics, aspects and advantages of the invention will emerge even more clearly on reading the description and the example that follows.

In the text herein below, and unless otherwise indicated, the limits of a range of values are included in that range, in particular in the expressions "between" and "ranging from ... to ...".

Moreover, the expression "at least one" used in the present description is equivalent to the expression "one or more".

The term "dispersion" encompasses the concept of suspension and emulsions.

### Method for preparing the nanosystem vehicle

As intended herein, the term "comprising" has the meaning of "including" or "containing", which means that when an object "comprises" one or several elements, other elements than those mentioned may also be included in the object. In contrast, when an object is said to "consist of' one or several elements, the object is limited to the listed elements and cannot include other elements than those mentioned.

As previously detailed, the present invention pertains to a method for preparing a nanosystem vehicle encapsulating one or more active ingredients, comprising:
i) bringing one or more amphiphilic compounds and one or more active ingredients into contact with an aqueous medium in a frozen state, preferably ice,
ii) cryomilling said amphiphilic compounds, said active ingredients and said aqueous medium to obtain a solid cryomilled mixture,
iii) thawing said solid cryomilled mixture until forming said nanosystem vehicle.

The aqueous medium, preferably water, is preferentially cooled down until freezing before one or more amphiphilic compounds and one or more active ingredients are brought into contact with said frozen aqueous medium.

In other words, the method preferably comprises cooling of an aqueous medium, especially water, until freezing before bringing one or more amphiphilic compounds and one or more active ingredients into contact with said aqueous medium in a frozen state.

Preferably, water is turned into ice before being brought into contact with one or more amphiphilic compounds and one or more active ingredients.

In one embodiment, the aqueous medium, preferably water, is cooled until it freezes at a temperature equal to or lower than 0°C.

In one embodiment, the aqueous medium, preferably water, is cooled until it freezes at a temperature equal to or lower than -10°C.

In one embodiment, the aqueous medium, preferably water, is cooled until it freezes at a temperature equal to or lower than -20°C.

In one embodiment, the aqueous medium, preferably water, is cooled until it freezes at a temperature equal to or lower than -30°C.

In one embodiment, the aqueous medium, preferably water, is cooled until it freezes at a temperature equal to or lower than -40°C.

In one embodiment, the aqueous medium, preferably water, is cooled until it freezes at a temperature equal to or lower than -50°C.

In one embodiment, the aqueous medium, preferably water, is cooled until it freezes at a temperature equal to or lower than -60°C.

In one embodiment, the aqueous medium, preferably water, is cooled until it freezes at a temperature equal to or lower than -70°C.

In one embodiment, the aqueous medium, preferably water, is cooled until it freezes at a temperature equal to or lower than -80°C.

In one embodiment, the aqueous medium, preferably water, is cooled until it freezes at a temperature equal to or lower than -90°C.

In one embodiment, the aqueous medium, preferably water, is cooled until it freezes at a temperature equal to or lower than -100°C.

In one embodiment, the aqueous medium, preferably water, is cooled until it freezes at a temperature equal to or lower than -110°C.

In one embodiment, the aqueous medium, preferably water, is cooled until it freezes at a temperature equal to or lower than -120°C.

In one embodiment, the aqueous medium, preferably water, is cooled until it freezes at a temperature equal to or lower than -130°C.

In one embodiment, the aqueous medium, preferably water, is cooled until it freezes at a temperature equal to or lower than -140°C.

In one embodiment, the aqueous medium, preferably water, is cooled until it freezes at a temperature equal to or lower than -150°C.

In one embodiment, the aqueous medium, preferably water, is cooled until it freezes at a temperature equal to or lower than -180°C.

The aqueous medium, preferentially water, is preferably cooled down at a cryogenics temperature to be instantaneously frozen in order to avoid premature swelling of the amphiphilic compounds, especially surfactants, when said compounds are brought into contact with said aqueous medium.

According to the present invention, the expression "cryogenics temperature" stands for a temperature that is equal to or lower than -150°C, especially lower than - 180°C.

In other words, the aqueous medium, preferentially water, is advantageously cooled down at a temperature equal to or lower than -150°C, more preferably at a temperature equal to or lower than -180°C.

Preferably, the aqueous medium, preferentially water, is advantageously cooled down with a cryogenic fluid.

According to the present invention, the feature "cryogenic fluid" refers to a fluid whose boiling point at atmospheric pressure is equal to or lower than -150°C. In particular, cryogenic fluid refers to a liquified gas that is kept in its liquid state at a temperature equal or lower than -150°C, especially lower than -180°C.

Cryogenic fluid can be selected from the group consisting of liquid nitrogen or liquid argon, preferably liquid nitrogen.

Preferably, the aqueous medium, preferentially water, is cooled down at a temperature equal to or lower than -150°C, more preferably at a temperature equal to or lower than -180°C, with a cryogenic fluid, especially liquid nitrogen.

Preferably, the aqueous medium is water that is cooled down with a cryogenic fluid, especially liquid nitrogen, in order to be frozen.

According to a preferential embodiment, the method comprises cooling a vial in a cryogenic fluid cooled environment, preferably in an environment cooled by liquid nitrogen, and adding water in the vial in order to be instantly frozen.

As previously mentioned, the method comprises bringing one or more amphiphilic compounds and one or more active ingredients into contact with said aqueous medium in a frozen state.

In other words, one or more amphiphilic compounds and one or more active ingredients can be either in a liquid or solid state and are added to the frozen aqueous medium, especially frozen water in order to be cryomilled.

In other words, the method comprises contacting one or more amphiphilic compounds, one or more active ingredients and said aqueous medium in a frozen state.

The amphiphilic compounds can be present at a concentration ranging from 0,01 to 80% by weight, preferably from 0,01 to 60% by weight, relative to the total weight mixture of the amphiphilic compounds and the active ingredients.

The active ingredients can be present at a concentration ranging from 0,01 to 80% by weight, preferably from 0,01 to 60% by weight, preferably from 0,01 to 10% by weight relative to the total weight mixture of the amphiphilic compounds and the active ingredients.

In one embodiment, one or more amphiphilic compounds and one or more active ingredients are brought into contact with the frozen aqueous medium at a temperature equal to or lower than 0°C.

In one embodiment, one or more amphiphilic compounds and one or more active ingredients are brought into contact with the frozen aqueous medium at a temperature equal to or lower than -10°C.

In one embodiment, one or more amphiphilic compounds and one or more active ingredients are brought into contact with the frozen aqueous medium at a temperature equal to or lower than -20°C.

In one embodiment, one or more amphiphilic compounds and one or more active ingredients are brought into contact with the frozen aqueous medium at a temperature equal to or lower than -30°C.

In one embodiment, one or more amphiphilic compounds and one or more active ingredients are brought into contact with the frozen aqueous medium at a temperature equal to or lower than -40°C.

In one embodiment, one or more amphiphilic compounds and one or more active ingredients are brought into contact with the frozen aqueous medium at a temperature equal to or lower than -50°C.

In one embodiment, one or more amphiphilic compounds and one or more active ingredients are brought into contact with the frozen aqueous medium at a temperature equal to or lower than -60°C.

In one embodiment, one or more amphiphilic compounds and one or more active ingredients are brought into contact with the frozen aqueous medium at a temperature equal to or lower than -70°C.

In one embodiment, one or more amphiphilic compounds and one or more active ingredients are brought into contact with the frozen aqueous medium at a temperature equal to or lower than -80°C.

In one embodiment, one or more amphiphilic compounds and one or more active ingredients are brought into contact with the frozen aqueous medium at a temperature equal to or lower than -90°C.

In one embodiment, one or more amphiphilic compounds and one or more active ingredients are brought into contact with the frozen aqueous medium at a temperature equal to or lower than -100°C.

In one embodiment, one or more amphiphilic compounds and one or more active ingredients are brought into contact with the frozen aqueous medium at a temperature equal to or lower than -110°C.

In one embodiment, one or more amphiphilic compounds and one or more active ingredients are brought into contact with the frozen aqueous medium at a temperature equal to or lower than -120°C.

In one embodiment, one or more amphiphilic compounds and one or more active ingredients are brought into contact with the frozen aqueous medium at a temperature equal to or lower than -130°C.

In one embodiment, one or more amphiphilic compounds and one or more active ingredients are brought into contact with the frozen aqueous medium at a temperature equal to or lower than -140°C.

In one embodiment, one or more amphiphilic compounds and one or more active ingredients are brought into contact with the frozen aqueous medium at a temperature equal to or lower than -150°C.

In one embodiment, one or more amphiphilic compounds and one or more active ingredients are brought into contact with the frozen aqueous medium at a temperature equal to or lower than -180°C.

Preferably, one or more amphiphilic compounds and one or more active ingredients are brought into contact with the frozen aqueous medium at a temperature equal to or lower than 0°C, preferably at a temperature equal to or lower than -150°C, more preferably at a temperature equal to or lower than -180°C.

The amphiphilic compound can be selected from the group consisting of polymers, surfactants, lipids and mixtures thereof, especially lipids, surfactants, and mixtures thereof, more especially in the group consisting of lipids.

The amphiphilic compound may be an amphiphilic lipid selected from the group consisting of phospholipids, synthetic phospholipids, glycerolipids, sphingolipids, glycosphingolipids and mixtures thereof.

The amphiphilic compound is preferably an amphiphilic lipid selected from the group consisting of phospholipids.

The phospholipids can be selected from the group consisting of lecithins (phosphatidyl-choline), phosphatidyl-ethanolamines, phosphatidyl-serines and phosphatidyl-inositol and mixtures thereof, preferably from lecithins, phosphatidyl-ethanolamines and mixture thereof, even more preferably from lecithins.

The amphiphilic compound may also be an amphiphilic polymer selected from the group consisting of amphiphilic block polymers or graft polymer, preferably amphiphilic block polymers.

The amphiphilic block polymers can be a di-block amphiphilic polymer, preferably a non-ionic di-block amphiphilic polymer.

In particular, the amphiphilic block polymer can comprise a hydrophilic block and a hydrophobic block linked with each other.

The hydrophilic block may be selected from the group consisting of polyvinyl alcohol, polyalkylene glycol, polyvinyl pyrrolidone, polyacrylamide and derivatives thereof. Preferably, the hydrophilic block is selected from the group consisting of polyalkylene glycol.

The hydrophobic block may be selected from the group consisting of polyester, polyanhydride, polyamino acid, polyorthoester, polyphosphazine and derivatives thereof.

The amphiphilic compound may also be an amphiphilic surfactant, preferably a polymeric amphiphilic surfactant.

According to the present invention, the term "polymeric amphiphilic surfactant" means the surfactant comprises a part in its structure that is polymeric.

The amphiphilic compound may be a non-ionic amphiphilic surfactant preferably selected from the group consisting of sorbitol esters of C₈-C₂₄ fatty acids, esters of C₈-C₂₄ fatty acids and ethoxylated sorbitan containing from 4 to 40 ethylene oxide, oxyethylenated C₈-C₂₄ alcohol, esters of tocopherol and polyalkylene glycol and mixtures thereof.

Preferably, the amphiphilic compound is a non-ionic amphiphilic surfactant selected from esters of C₈-C₂₄ fatty acids and ethoxylated sorbitan containing from 4 to 40 ethylene oxide, more preferably from esters of C₁₂-C₁₈ fatty acids and ethoxylated sorbitan containing from 4 to 40 ethylene oxide, esters of tocopherol and polyalkylene glycol, preferably esters of tocopherol and polyethylene glycol, and mixtures thereof.

According to the present invention, the term tocopherol may include α-tocopherol, D-α-tocopherol, β-tocopherol, γ-tocopherol and δ-tocopherol, in particular D-α-tocopherol.

Preferably, esters of tocopherol and polyalkylene glycol are obtained from esterification of acid esters of tocopherol and polyalkylene glycol.

Acid esters of tocopherol can be chosen among the group consisting of tocopheryl acid succinates, tocopheryl acid citraconates, tocopheryl acid methyl citraconate, tocopheryl acid itaconate, tocopheryl acid maleate.

Preferably, esters of tocopherol and polyalkylene glycol are chosen among the group consisting of tocopherol polyethylene glycol succinate (TGPS), tocopherol sebacate polyethylene glycol, tocopherol citraconate polyethylene glycol, tocopherol methyl citraconate polyethylene glycol, tocopherol itaconate polyethylene glycol, tocopherol maleate polyethylene glycol and mixtures thereof.

Preferably, the non-ionic amphiphilic surfactant is chosen among tocopherol polyethylene glycol succinate (TGPS) and its derivatives, especially D-α-tocopherol polyethylene glycol succinate (TGPS), more specifically -α-tocopherol polyethylene glycol succinate having 1000 oxide ethylene units.

The non-ionic amphiphilic surfactant may also be chosen among polycondensates including polyethylene glycol/polypropylene glycol/polyethylene glycol triblock polycondensate, such as the ethylene oxide, propylene oxide and ethylene oxide condensate (98 OE/67 OP/98 OE) (MW: 12000), having the INCI name of Poloxamer 407, or the ethylene oxide/propylene oxide/ethylene oxide condensate (75 OE/30 OP/75 OE) (MW: 8350), having the INCI name of Poloxamer 188, or any other surfactant from poloxamer family.

Preferably, the amphiphilic compound can be chosen from the group consisting of phospholipids, such as lecithins and phosphatidyl-ethanolamines, non-ionic amphiphilic block polymers, preferably non-ionic amphiphilic di-block polymers, non-ionic surfactant chosen from the group consisting of sorbitol esters of C₈-C₂₄ fatty acids, esters of C₈-C₂₄ fatty acids and ethoxylated sorbitan containing from 4 to 40 ethylene oxide, esters of tocopherol and polyalkylene glycol, polycondensates, and mixtures thereof.

More preferably, the amphiphilic compound can be chosen from the group consisting of phospholipids, such as lecithins, non-ionic amphiphilic di-block polymers, esters of C₈-C₂₄, especially C₁₂-C₁₈, fatty acids and ethoxylated sorbitan containing from 4 to 40 ethylene oxide, esters of tocopherol and polyalkylene glycol, polycondensates, especially from the group consisting of phospholipids esters of tocopherol and polyalkylene glycol, and polycondensates.

Even more preferably, the amphiphilic compound can be chosen from the group consisting of lecithins and esters of tocopherol, polyalkylene glycol and polycondensates, such as the ethylene oxide, propylene oxide and ethylene oxide condensate.

The amphiphilic compound can be an amphiphilic lipid, preferably phospholipids, or a polymeric amphiphilic surfactant, preferably esters of tocopherol and polyalkylene glycol.

The active ingredients are preferentially selected from the group consisting of biological active ingredients, cosmetic active ingredients, dermatological active ingredients, agrochemical active ingredients and mixtures thereof, more preferably biological active ingredients, dermatological active ingredients and cosmetic ingredients, even more preferably biological active ingredients and cosmetic ingredients, especially biological active ingredients.

According to the present invention, the term "biological active ingredient" means a compound that affects biological processes in a way that has an impact on body functions.

The biological active ingredient can be selected from the group consisting of pharmaceutical active ingredients, enzymes, proteins and mixtures thereof, preferably from pharmaceutical active ingredients.

According to the present invention, the term "pharmaceutical active ingredient" means any substance or its salts thereof used in a finished pharmaceutical product, intended to provide a pharmacological activity or to otherwise have direct effect in the diagnosis, cure, mitigation, treatment or prevention of disease, or to have direct effect in restoring, correcting or modifying physiological functions in human beings.

The pharmaceutical active ingredients may be hydrophilic or hydrophobic.

The pharmaceutical active ingredient can be chosen from the group consisting of any hydrophilic, lipophilic and amphiphilic pharmaceutically active ingredients either alone or in combination.

Preferably, the active ingredients are selected from lipophilic active ingredients, especially lipophilic biological active ingredients, more preferably lipophilic pharmaceutical active ingredients.

The pharmaceutical active ingredients may be lipophilic and/or hydrophilic molecules and chosen among any category of drugs, preferably immunosuppressive agents, anticancer agents, nucleic acids drugs and antifungal agents.

The pharmaceutical active ingredients may be chosen among the group consisting of cyclosporine, voriconazole, natamycin, itraconazole and their pharmaceutically acceptable salts, especially cyclosporine, voriconazole and their pharmaceutically acceptable salts, more preferably cyclosporine.

The pharmaceutical active ingredients may be chosen among the group consisting of cyclic polypeptide, preferably cyclosporine.

Preferably, the amphiphilic compound can be an amphiphilic lipid, preferably phospholipids, or a polymeric amphiphilic surfactant, preferably esters of tocopherol and polyalkylene glycol, and the active ingredient can be a pharmaceutical active ingredient especially chosen among the group consisting of cyclic polypeptide, preferably cyclosporine.

Even more preferably, the amphiphilic compound can be a polymeric amphiphilic surfactant and the active ingredient can be a pharmaceutical active ingredient chosen among the group consisting of cyclic polypeptide, especially cyclosporine.

As previously mentioned, the method further comprises cryomilling said amphiphilic compounds, said active ingredients and said aqueous medium to obtain a solid cryomilled mixture.

In particular, cryomilling allows to mill the aforementioned amphiphilic compounds, active ingredients and said frozen aqueous medium to form a solid homogenous mixture reduced into nanometric sized particles.

In particular, such nanometric sized particles are free from each other.

Indeed, cryomilling especially relies on impact and attrition to reduce the size of the mixture of the aforementioned amphiphilic compounds, active ingredients and frozen aqueous medium into nanometric sized particles. In each nanometric sized particles, said amphiphilic compounds, active ingredients and frozen aqueous medium form a homogenous mixture.

In accordance with the present invention, cryomilling can take place within a high energy mill, such as an attritor mill, for example an attritor mill having metallic or ceramic balls.

An example of high energy mill in order to perform cryomilling can be SPEX^{®} SamplePrep cryomill, Retsch^{®} cryomill, AIR PRODUCTS cryomill.

Preferably, cryomilling is performed at a cryogenics temperature. In other words, the composition is milled at a cryogenics temperature.

Preferably, cryomilling is performed at a cryogenics temperature. In other words, the composition is milled at a temperature equal to or lower than -150°C, especially lower than -180°C.

Preferably, the amphiphilic compounds, active ingredients and frozen aqueous medium are milled in a cryogenic fluid cooled environment.

According to the present invention, the expression "a cryogenic fluid cooled environment" means that the amphiphilic compounds, active ingredients and frozen aqueous medium are in an environment that is cooled by a cryogenic fluid as previously defined. In other words, cryomilling preferably occurs in an environment cooled by a cryogenic fluid as previously defined.

Cryogenic fluid can be selected from the group consisting of liquid nitrogen or liquid argon, preferably liquid nitrogen.

Preferably, cryomilling is performed in a liquid nitrogen cooled environment. In other words, the amphiphilic compounds, active ingredients and frozen aqueous medium are immersed in liquid nitrogen during milling.

Advantageously, cryomilling is performed in an attritor mill in a cryogenic fluid, especially in a liquid nitrogen, cooled environment.

Cryomilling can be carried out for a period of time ranging from 1 min to 360 minutes, preferably from 5 minutes to 60 minutes.

As previously mentioned, the method further comprises thawing the solid cryomilled mixture, preferably at a temperature ranging from 20°C to 80°C, preferably ranging from 20°C to 60°C, more preferably ranging from 20°C to 50°C, even more preferably ranging from 20°C to 40°C and especially ranging from 20°C to 30°C.

In particular, according to the present invention, during thawing, the aqueous medium melts and hydrates the mixture of amphiphilic compounds and active ingredients.

As a result of thawing, a nanosystem vehicle encapsulating said active ingredients is formed, advantageously instantly formed.

According to a preferential embodiment, the method comprises:
i) cooling of an aqueous medium, preferably water, until freezing, preferably at a temperature equal to or lower than 0°C, more preferably at a temperature equal to or lower than -150°C, even more preferably at a temperature equal to or lower than -180°C,
ii) bringing into contact one or more amphiphilic compounds and one or more active ingredients, preferably biological active ingredients, with said aqueous medium in a frozen state, preferably at a temperature equal to or lower than 0°C, more preferably at a temperature equal to or lower than -150°C, even more preferably at a temperature equal to or lower than -180°C,
iii) cryomilling said amphiphilic compounds, said active ingredients and said aqueous medium to obtain a solid cryomilled mixture,
iv) thawing said solid cryomilled mixture until forming said nanosystem vehicle.

According to a preferential embodiment, the method comprises:
i) cooling a vial in a cryogenic fluid cooled environment, preferably in an environment cooled by liquid nitrogen,
ii) adding an aqueous medium, preferably water, in the vial in order to be frozen,
iii) adding one or more amphiphilic compounds, as previously detailed, and one or more active ingredients, as previously detailed, preferably biological active ingredients, especially pharmaceutical active ingredients in said vial,
iv) cryomilling said amphiphilic compounds, said active ingredients and the frozen aqueous medium, especially frozen water, to obtain a solid cryomilled mixture,
v) thawing said solid cryomilled mixture until forming a nanosystem vehicle encapsulating said active ingredients.

In the aforementioned preferential embodiment of the method, the vial is still placed in a cryogenic fluid cooled environment. In other words, steps (ii) and (iii) are carried out in a cryogenic fluid cooled environment, preferably liquid nitrogen.

The nanosystem vehicle prepared according to the aforementioned method can be a vesicular nanosystem.

The vesicular nanosystem is preferably selected from the group consisting of micelles, liposomes, niosomes, sphingosomes, transferosomes and ethosomes.

Preferably, the vesicular nanosystem can be selected from the group consisting of micelles, liposomes and niosomes, more preferably from micelles and liposomes, even more preferably from polymeric micelles and liposomes.

In case when multilamellar vesicles, especially in bilayer vesicles structures such as liposomes, are obtained, a conversion into unilamellar vesicles can be implemented, particularly by extrusion or ultrasonication.

Preferably, when multilamellar liposomes are obtained after thawing, a conversion into unilamellar liposomes can advantageously be implemented.

Converting said multilamellar vesicles into unilamellar vesicles can be performed by extrusion or not, preferably not by extrusion.

In particular, converting multilamellar vesicles into unilamellar vesicles can be performed by ultrasonication.

In one embodiment, the method according to the present invention can further comprise adding one or more excipients to the nanosystem vehicle after its formation.

The excipient may be chosen in among the group consisting of pH modifiers, lubricants, preservatives, diluents, osmosity modulators, stabilizers.

In an embodiment, the aforementioned nanosystem vehicle can comprise at most between 0.05 and 70% by weight of excipients, preferably at most between 0.1 and 50% by weight of excipients, relative to the weight of the nanosystem.

The method according to the present invention is particularly free of water-soluble solutes or wetting agents, such as glucose.

Preferably, the aqueous medium is devoid of water-soluble solutes or wetting agents, such as glucose.

According to the present invention, the term "water-soluble solutes" refers to fillers selected from the group consisting of water-soluble salts, such as sodium chloride, sugar alcohols, such as mannitol, sorbitol, microcrystalline cellulose, lactose, polysaccharides such as sucrose, glucose, mannose, dextrans, starch saccharification products, water soluble polymers such as polyethylene glycol, polyvinylpyrrolidone and mixtures thereof.

In other words, the method according to the present invention is particularly free of fillers selected from the group consisting of water-soluble salts, such as sodium chloride, sugar alcohols, such as mannitol, sorbitol, microcrystalline cellulose, lactose, monosaccharides and polysaccharides such as sucrose, glucose, mannose, dextrans, starch saccharification products, water soluble polymers such as polyethylene glycol, polyvinylpyrrolidone and mixtures thereof.

Preferably, the method according to the present invention does not involve the implementation of glucose.

The method according to the present invention is preferably free of organic solvent.

According to the present invention, the feature "free of organic solvent" means that the method does not involve the implementation of any organic solvent.

In particular, the method according to the present invention is a solvent free process, i.e. the method does not involve the implementation of any organic solvent at any of its steps.

In an embodiment, organic solvent can be chosen from the group consisting of alcohols like methanol, ethanol, propanol, acetone, chloroform, ethers, tetrahydrofuran, dichloromethane.

Preferably, the method is free of organic solvents and of water-soluble solutes or wetting agents, such as glucose.

In one embodiment, the method according to the present invention can optionally comprise bringing one or more amphiphilic compounds, one or more active ingredients and one or more lipids that are different from the lipids previously described into contact with said aqueous medium in a frozen state.

In other words, the method comprises bringing one or more amphiphilic compounds, one or more active ingredients, and optionally one or more lipids that are different from the lipids previously described, into contact with said aqueous medium in a frozen state.

In that respect, lipids can be chosen in the group consisting of sterols, preferably cholesterol.

### Nanosystem vehicles

The invention is also directed to a nanosystem vehicle, obtainable from the method previously defined, for its use in a therapeutic treatment and/or *in vivo* diagnosis method, especially therapeutic treatment, comprising one or more biological active ingredients as previously disclosed, preferably chosen in the group consisting of pharmaceutical active ingredients.

In other words, the invention also relates to a nanosystem vehicle as previously defined for use in a therapeutic treatment and/or *in vivo* diagnosis method, especially in a therapeutic treatment, comprising one or more biological active ingredients as previously disclosed, preferably chosen in the group consisting of pharmaceutical active ingredients.

The nanosystem vehicle according to the invention is free of water-soluble solute or a wetting agent, such as glucose.

Preferably, the invention relates to a liposome vehicle, for its use in a therapeutic treatment and/or *in vivo* diagnosis method, especially therapeutic treatment, comprising one or more biological active ingredients as previously disclosed, preferably chosen in the group consisting of pharmaceutical active ingredients.

In other words, the invention also relates to a liposome vehicle, for use in a therapeutic treatment and/or *in vivo* diagnosis method, especially in a therapeutic treatment, comprising one or more biological active ingredients as previously disclosed, preferably chosen in the group consisting of pharmaceutical active ingredients

According to this embodiment, the amphiphilic compound is preferably selected among the group consisting of lipids.

Preferably, the invention relates to a micelle vehicle, for its use in a therapeutic treatment and/or *in vivo* diagnosis method, especially therapeutic treatment, comprising one or more biological active ingredients as previously disclosed, preferably chosen in the group consisting of pharmaceutical active ingredients as preferably defined.

In other words, the invention also relates a micelle vehicle, for use in a therapeutic treatment and/or *in vivo* diagnosis method, especially in a therapeutic treatment, comprising one or more biological active ingredients as previously disclosed, preferably chosen in the group consisting of pharmaceutical active ingredients as preferably defined.

According to this embodiment, the amphiphilic compound is preferably selected among the group consisting of surfactants.

Preferably, the pharmaceutical active ingredient is lipophilic and/or hydrophilic molecule may be chosen among any category of drugs, more preferably immunosuppressive agents, anti-inflammatory, anticancer agents and antifungal agents, preferably anticancer agents.

Another subject of the present invention pertains to the use of a nanosystem vehicle, obtainable from the method previously defined, in order to impart cosmetic properties to human keratin materials, preferably to skin, comprising one or more cosmetic active ingredients.

The following examples are given as illustrations of the present invention.

### Example 1: Micelles formulation of cyclosporine

A vial was placed in an environment cooled by liquid nitrogen and water was then incorporated into said vial in order to be instantly frozen.

Non-ionic polymeric surfactant (TPGS) and cyclosporine (CsA) were then brought into contact with the frozen water in the vial. The vial is still placed in said environment cooled by liquid nitrogen.

Non-ionic polymeric surfactant (TPGS), cyclosporine (CsA) and the frozen water were then cryomilled using SamplePrep^{®} freezer mill 6770 in order to form a solid homogenous mixture.

The vial was then placed in a hot water bath to be thawed. Micelles encapsulating cyclosporine were formed upon thawing.

The characterization parameters of the polymeric micelles are given in Table 1.

**Table 1: Micelle formulations**

| **TPGS concentration** | **CsA targeted concentration** | **CsA encapsulated concentration** | **Entrapment efficiency %** | **Particle size (Zav, nm)** |
|---|---|---|---|---|
| 20 mg/mL | 3 mg/mL | 3 mg/mL | 100.01± 2.1 | 11.51 |

The Z average of the particle size corresponds to the intensity weighted mean hydrodynamic size of the ensemble collection of particles measured by dynamic light scattering (DLS).

The CsA content and entrapment efficiency have been determined by using Acquity^{®} Core UPLC^{®} system equipped with Xevo^{®} TQ-MS tandem quadrupole detector system.

### Example 2: Liposomes formulation of cyclosporine

A vial was placed in an environment cooled by liquid nitrogen and water was then incorporated into said vial in order to be instantly frozen.

Lipoid S-100 (soyabean phophatidylcholine) and cyclosporine (CsA) were then brought into contact with the frozen water in the vial. The vial is still placed in said environment cooled by liquid nitrogen.

Lipoid S-100, cyclosporine (CsA) and the frozen water were then cryomilled using SamplePrep^{®} freezer mill 6770 in order to form a solid homogenous mixture.

The vial was then placed in a hot water bath to be thawed. Liposomes encapsulating cyclosporine were formed upon thawing.

The characterization parameters of the polymeric micelles are given in Table 2.

**Table 2: Liposome formulations**

| **Lipoid S100 concentration** | **CsA targeted concentration** | **CsA encapsulated concentration** | **Entrapment efficiency %** | **Particle size (Zav, nm)** |
|---|---|---|---|---|
| 60 mg/mL | 3 mg/mL | 2.95 mg/mL | 98.33± 4.5 | -1.3 |

The CsA content and entrapment efficiency have been determined by using Acquity Core UPLC^{®} system equipped with Xevo^{®} TQ-MS tandem quadrupole detector system. Malvern Zetasizer was used to determine particle size of the liposome.

## Claims

1. Method for preparing a nanosystem vehicle encapsulating one or more active ingredients, comprising:
i) bringing one or more amphiphilic compounds and one or more active ingredients into contact with an aqueous medium in a frozen state,
ii) cryomilling said amphiphilic compounds, said active ingredients and said aqueous medium to obtain a solid cryomilled mixture,
iii) thawing said solid cryomilled mixture until forming said nanosystem.

2. Method according to Claim 1, **characterized in that** the aqueous medium is cooled down until freezing before being brought into contact with said amphiphilic compounds and said active ingredients.

3. Method according to Claim 1 or 2, **characterized in that** the aqueous medium is cooled down at a temperature equal to or lower than 0°C.

4. Method according to any of Claims 1 to 3, **characterized in that** the aqueous medium is cooled down at a cryogenics temperature, preferably at a temperature equal to or lower than -150°C.

5. Method according to any of preceding claims, **characterized in that** the aqueous medium is cooled down with a cryogenic fluid, preferably with liquid nitrogen or liquid argon.

6. Method according to any of preceding claims, **characterized in that** the amphiphilic compound is selected from the group consisting of polymers, surfactants, lipids and mixtures thereof, preferably from lipids, polymeric surfactants and mixtures thereof, even more preferably from lipids.

7. Method according to any of preceding claims, **characterized in that** said amphiphilic compound is an amphiphilic lipid selected from the group consisting of phospholipids, synthetic phospholipids, glycerolipids, sphingolipids, glycosphingolipids and mixtures thereof, preferably phospholipids.

8. Method according to any of preceding claims, **characterized in that** the amphiphilic compound is selected from the group consisting of phospholipids, amphiphilic block and/or graft copolymers, preferably non-ionic amphiphilic di-block polymers, non-ionic surfactant chosen from the group consisting of sorbitol esters of C₈-C₂₄ fatty acids, esters of C₈-C₂₄ fatty acids and ethoxylated sorbitan containing from 4 to 40 ethylene oxide, esters of tocopherol and polyalkylene glycol and mixtures thereof.

9. Method according to any of preceding claims, **characterized in that** the active ingredients are selected from the group consisting of biological active ingredients, cosmetic active ingredients, dermatological active ingredients and mixtures thereof, especially biological active ingredients.

10. Method according to Claim 9, **characterized in that** the biological active ingredients are chosen from pharmaceutical active ingredients, especially cyclic polypeptide.

11. Method according to any of preceding claims, **characterized in that** thawing is carried out at a temperature ranging from 20°C to 80°C, more preferably ranging 20°C to 50°C, even more preferably ranging from 20°C to 30°C.

12. Method according to any of preceding claims, **characterized in that** said nanosystem vehicle is a vesicular nanosystem selected from the group consisting of micelles, liposomes, niosomes, sphingosomes, transferosomes and ethosomes, preferably from micelles and liposomes.

13. Method according to Claim 12, **characterized in that** when multilamellar vesicles are obtained, a conversion into unilamellar vesicles is implemented by extrusion or ultrasonication.

14. Method according to any of preceding claims, **characterized in that** said method is free of water-soluble solutes or wetting agents, preferably glucose.
